(19) Europäisches Patentamt
European Patent Office
Office européen des brevets

(11) Publication number: **0 033 585**
**B1**

(12) # EUROPEAN PATENT SPECIFICATION

(45) Date of publication of patent specification: **20.04.83**

(51) Int. Cl.³: **C 07 C 89/00,**
C 07 C 91/22, C 07 C 97/10

(21) Application number: **81300101.3**

(22) Date of filing: **09.01.81**

(54) **A process for the preparation of a benzhydrol derivative and a novel intermediate for use therein.**

(30) Priority: **11.01.80 FI 800076**

(43) Date of publication of application:
**12.08.81 Bulletin 81/32**

(45) Publication of the grant of the patent:
**20.04.83 Bulletin 83/16**

(84) Designated Contracting States:
**AT BE CH DE FR GB IT LI LU NL SE**

(56) References cited:
**CH - A - 562 195**
**US - A - 3 487 153**
**US - A - 4 208 349**

**Chemical Abstracts vol. 94, no. 11, 16 March 1981**
**Columbus, Ohio, USA**
**FARMA-LEPORI "2-(N-2-Hydroxyethylmethylaminomethyl)benzhydrol"**
**page 690, column 2, abstract no. 83757s**

(73) Proprietor: **Farmos-Yhtymä Oy**
**P.O. Box 425**
**SF-20101 Turku 10 (FI)**

(72) Inventor: **Saxlund, Raimo Antero**
**Koskitie 26 E 15-16**
**SF-95100 Oulu 50 (FI)**
Inventor: **Koskenniska, Lasse Antero**
**Liistetie 4 A 2**
**SF-90650 Oulu 65 (FI)**

(74) Representative: **Collier, Jeremy Austin Grey et al,**
**J.A.Kemp & Co. 14, South Square Gray's Inn**
**London WC1R 5EU (GB)**

**EP 0 033 585 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# A process for the preparation of a benzhydrol derivative and a novel intermediate for use therein

This invention relates to the production of 2-{[N-(2-hydroxyethyl)-N-methyl]amino}-methylbenzhydrol having the formula:

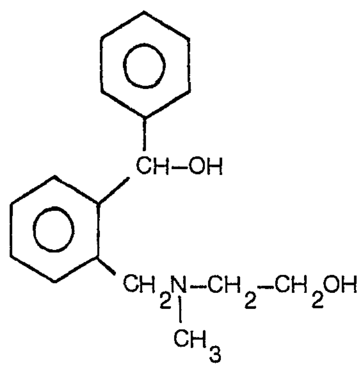

I

This compound is useful as an intermediate in producing the pharmacologically valuable 3,4,5,6-tetrahydro-5-methyl-1-phenyl-1H-2,5-benzoxazocinehydrochloride, or nefopam, which is used, e.g. as a muscle relaxant, an analgesic or antidepressant drug.

Processes for producing the compound of formula I are already known. For instance, according to German Patent 1,620,198, phthalic aldehyde is used as a starting material. According to the German Patent, the phthalic aldehyde is reacted with a Grignard reagent, phenylmagnesiumbromide, and an N-substituted aminoalcohol is coupled to the reaction mixture, to produce a product of formula:

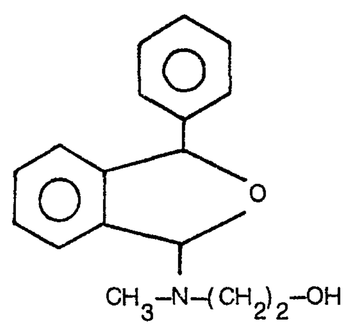

II

This product is catalytically hydrogenated with the aid of Pd/C, Pt or Raney-Ni, and a product of formula I is obtained.

In another method, according to the German Patent 1,620,198, o-benzoylbenzoic acid is used as a starting material, which is converted by means of thionylchloride into an acid chloride. To this acid chloride is then coupled methylethanolamine, and N-(2-hydroxyethyl)-N-methyl-o-benzoylbenzamide is obtained as an intermediate, which is reduced using $LiAlH_4$ and an end-product of formula I is produced.

According to United States Patent 3,487,153 o-benzoylbenzoic acid amide is used as starting material to produce the intermediate. With the aid of thionylchloride the corresponding acid chloride is formed, which is allowed to react with N-methyl-2-aminoethanol. The so-produced N-(2-hydroxyethyl)-N-methyl-o-benzoylbenzamide is reduced with $LiAlH_4$ to 2{[N-(2-hydroxyethyl)-N-methyl]amino}-methylbenzhydrol.

According to German Offenlegungschrift 2,834,312 o-benzoylbenzoic acid is used as a starting material, which is allowed to react with phosphorus trichloride in dichloroethane. The acid chloride formed is allowed to react with triethylamine and N-methyl-2-hydroxyethylamine, after which N-(2-hydroxyethyl)-N-methyl-o-benzoylbenzamide is formed. This compound is treated with phosphorus trichloride (at pH = 7.0) and N-(2-chloroethyl)-N-methyl-o-benzoylbenzoic amide is obtained, which is then reduced with $NaBH_4$ in acetic acid. By these means 2-{[N-(2-hydroxyethyl)-N-methyl]-amino}-methylbenzhydrol is obtained.

According to Finnish Patent No. 54793, which corresponds to Canadian Patent 982,608, a compound of formula III is used as starting material, which is reduced with $NaBH_4$ to a corresponding benzhydrol derivative of formula IV, which is then allowed to react with an alkylamine to an $\alpha$-substituted 2-aminomethylbenzylalcohol of formula V. The abovementioned Patent does not concern either the preparation of nefopam or its intermediates.

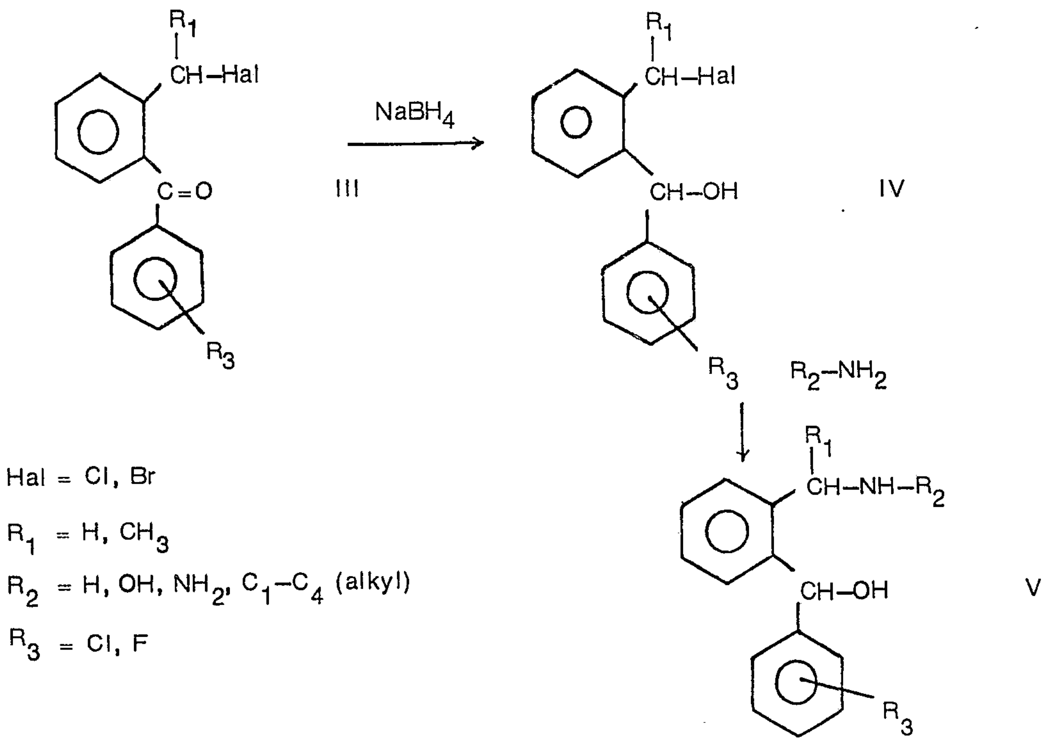


III IV V

Hal = Cl, Br

$R_1$ = H, $CH_3$

$R_2$ = H, OH, $NH_2$, $C_1$–$C_4$ (alkyl)

$R_3$ = Cl, F

When reviewing the abovementioned Patents, i.e. German Patent 1,620,198 and United States Patent 3,487,153, one can observe the disadvantage that catalytic hydrogenation with palladium on charcoal, platinum or Raney-Ni, or lithium aluminium hydride are to be used to reduce the starting materials. This latter reagent is expensive and reacts with water very intensely, so that even a little humidity in the working surroundings or in the solvents can cause a fire. Explosive hydrogen is also produced by the reaction. Grignard reactions and catalytic hydrogenations are technically difficult to perform on a large scale. Moreover, the price of o-phthalic aldehyde is high.

According to the method described in German Offenlegungschrift 2,834,312 the reducing of the amidecarbonyl group with sodium borohydride in acetic acid requires, however, great additional amounts or about 2—3 equivalents of sodium borohydride. The yield of the reaction is quite poor (about 50—55%) and the reaction time is long, so the production costs become high. Moreover, the number of synthetic reaction steps is high and the use of phosphorus trichloride especially on a production scale is difficult.

In the method according to the Finnish Patent 54793, which corresponds to the Canadian Patent 982,608, a benzophenone derivative (of formula III) is reduced with $NaBH_4$ to the corresponding benzhydrol derivative (formula IV). This compound is, however, unstable because of the methylene halogen group in *o*-position, especially when $R_1 = H$ in formula IV. On storing for only a short time hydrogenchloride gas is released and a very stable 5-ring ether is formed, which is useless. The use of this method on a large scale is therefore almost impossible, because the intermediate is impossible to isolate fast enough to obtain at least a reasonable amount of the end product.

The process of the present invention for producing 2{[N-(2-hydroxyethyl)-N-methyl]amino}-methylbenzhydrol reduces the abovementioned disadvantages. It is more economical and easy to perform, especially on a technical scale, than the before-mentioned methods. There are only two reaction steps in the new process, the first intermediate is a stable product and the reduction is performed by using only half an equivalent of sodium borohydride. Sodium borohydride is cheaper and easier to use than lithium aluminium hydride or catalytic hydrogenation.

In addition, in the process of invention, the formation of Schiffs bases has not been observed to occur between the amine and the carbonyl group of the benzophenone derivative, which could be possible, if primary amines, as the Finnish Patent 54783, were used. The yield, in the new process, is 95—96%.

The present invention provides a process for the preparation of 2-{[N-(2-hydroxyethyl)-N-methyl]-amino}-methylbenzhydrol (as such or as an acid addition salt) which comprises reacting 2-chloromethylbenzophenone with 2-methylaminoethanol to give 2-{[N-(2-hydroxyethyl)-N-methyl]-amino}-methylbenzophenone (as such or as a salt), and reducing the latter with sodium borohydride to give 2-{[N-2-(hydroxyethyl)-N-methyl]-amino}-methylbenzhydrol (as such or as an acid addition salt). The 2-chlorobenzophenone (of formula VI) is brought to react with methylethanolamine in the presence of e.g. sodium carbonate, and 2-{[N-(2-hydroxyethyl)-N-methyl]-amino}-methylbenzophenone (of formula VII)

is formed. This substance is then reduced with sodium borohydride to 2-{[N-(2-hydroxyethyl)-N-methyl]-amino}-methylbenzhydrol (of formula VIII), as shown below:

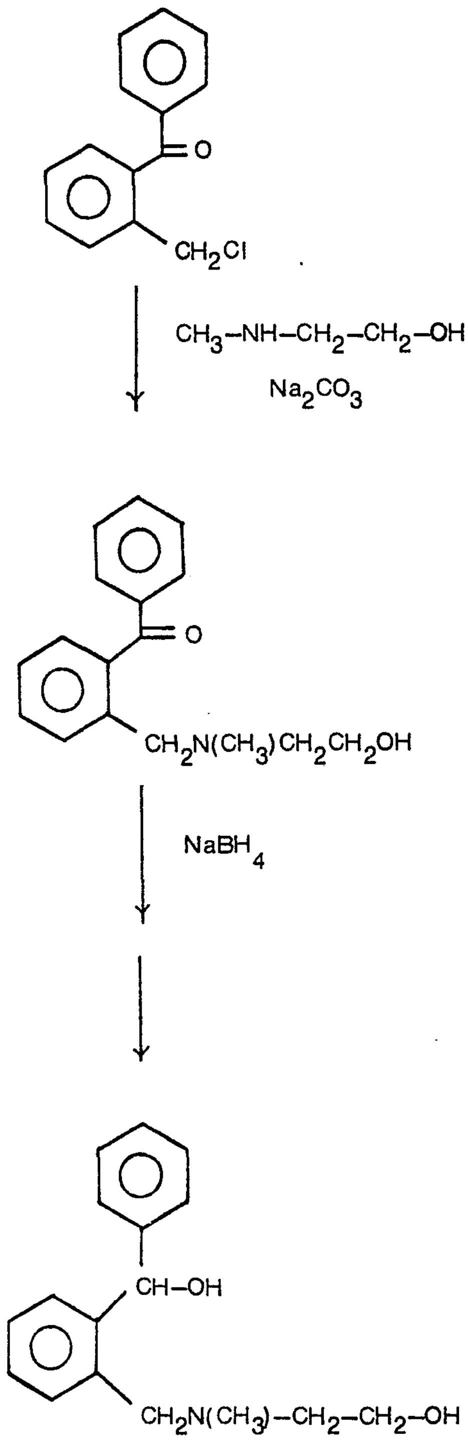


VI

VII

VIII

The starting material, 2-chloromethyl benzophenone, can be produced in known manner by halogenating the corresponding 2-methylbenzophenone (Monatshefte für Chemie *99*, 1990—2003, 1968) or 2-hydroxymethylbenzophenone, of which the former is commercially available and the latter can be produced in known manner from the phthalide (see British Patent 1,526,331). The compound of formula VII is new, and as such a feature of the invention.

The following Examples illustrate the invention.

## EXAMPLE 1

8.50 g (0.037 mol) 2-chloromethylbenzophenone is dissolved in 40 ml ethylalcohol, and 4.0 g sodium carbonate and 2.80 g (0.037 mol) 2-methylaminoethanol are added. The mixture is boiled for 3

hours and the salts formed are filtered off from the cooled solution. A pure reaction product is obtained when the ethanol is evaporated from the solution and the product is crystallized as a hydrochloride salt from a mixture of diethylether and alcohol. The yield is 10.7 g (95%) of 2-{[N-(2-hydroxyethyl)-N-methyl]-amino}-methylbenzophenone as a crystalline powder, m.p. 135—136°C.

This compound, as the free base, shows the following N M R spectrum (in $CDCl_3$ using T M S as internal reference): 7.8—7.1 (aromatic), 3.5 (singlet), 3.4 (triplet), about 2.6 (singlet), 2.3 (triplet), 1.9 (singlet). Its infra-red spectrum shows maxima at the following frequencies ($cm^{-1}$): 680, 720, 760, 910, 1010, 1060, 1140, 1230, 1260, 1300, 1430, 1560, 1580, 1640, 2760, 2920, 3030 and 3400.

EXAMPLE 2

10.0 g (0.033 mol) of the hydrochloride salt prepared in Example 1 are dissolved in a mixture comprising 15 ml water, 60 ml methanol and 3.5 g sodium hydroxide. To the mixture is added 0.65 g sodium borohydride and the solution is mixed for half an hour at room temperature.

The solution is acidified with concentrated hydrochloric acid and the methanol is evaporated in vacum. 40 ml of water is added, the pH of the water solution is adjusted with diluted sodium hydroxide solution to an alkaline reaction and the product is extracted into chloroform. The chloroform extracts are washed well with water, dried over sodium sulphate and evaporated to dryness. The product is separated by precipitating as a hydrochloride salt from a mixture of diethylether and ethylalcohol. The yield is 9.8 g (96%) of 2-{[N-(2-hydroxyethyl)-N-methyl]-amino}-methylbenzhydrol as a crystalline powder, m.p. 128—133°C.

**Claims for the Contracting States: BE CH DE FR GB IT LI LU NL SE**

1. A process for the preparation of 2-{[N-(2-hydroxyethyl)-N-methyl]-amino}-methylbenzhydrol (as such or as an acid addition salt) which comprises reacting 2-chloromethylbenzophenone with 2-methylaminoethanol to give 2-{[N-(2-hydroxyethyl)-N-methyl]-amino}-methylbenzophenone (as such or as a salt), and reducing the latter with sodium borohydride to give 2-{[N-(2-hydroxyethyl)-N-methyl]-amino}-methylbenzhydrol (as such or as an acid addition salt).

2. A process according to Claim 1 in which N-{[N-(2-hydroxyethyl)-N-methyl]amino}-methylbenzophenone is isolated in the form of its hydrochloride which is then reduced.

3. A process according to Claim 2 in which 0.065 part of sodium borohydride is used for each part by weight of 2-{[N-(2-hydroxyethyl)-N-methyl]-amino}-methylbenzophenone hydrochloride.

4. 2-{[N-(2-Hydroxyethyl)-N-methyl]-amino}-methylbenzophenone, and its acid addition salts.

5. A process for the preparation of a compound as claimed in Claim 4 which comprises reacting 2-methylamino-ethanol with 2-chloromethylbenzophenone.

**Claims for the Contracting State: AT**

1. A process for the preparation of 2-{[N-(2-hydroxyethyl)-N-methyl]-amino}-methylbenzhydrol (as such or as an acid addition salt) which comprises reacting 2-chloromethylbenzophenone with 2-methylamino-ethanol to give 2-{[N-(2-hydroxyethyl)-N-methyl]-amino}-methylbenzophenone (as such or as a salt), and reducing the latter with sodium borohydride to give 2-{[N-(2-hydroxyethyl)-N-methyl]-amino}-methylbenzhydrol (as such or as an acid addition salt).

2. A process according to claim 1 in which N-{[N-(2-hydroxyethyl)-N-methyl]amino}-methylbenzophenone is isolated in the form of its hydrochloride which is then reduced.

3. A process according to claim 2 in which 0.065 part of sodium borohydride is used for each part by weight of 2-{[N-(2-hydroxyethyl)-N-methyl]-amino}-methylbenzophenone hydrochloride.

4. A process for the preparation of 2-{[N-(2-hydroxyethyl)-N-methyl]-amino}-methylbenzophenone (as such or as an acid addition salt), which comprises reacting 2-methyl-amino-ethanol with 2-chloromethylbenzophenone.

**Revendications pour les Etats Contractants: BE CH LI DE FR GB IT LU NL SE**

1. Procédé de préparation du 2-{[N-(2-hydroxyéthyl)-N-méthyl]amino}-méthylbenzhydrol (tel quel ou sous la forme d'un sel d'addition acide) consistant à faire réagir la 2-chlorométhylbenzophénone sur le 2-méthylaminoéthanol pour donner la 2-{[N-(2-hydroxyéthyl)-N-méthyl]-amino}-méthylbenzophénone (telle quelle ou sous la forme d'un sel) et à réduire cette dernière par du borhydrure de sodium pour donner le 2-{[N-(2-hydroxyéthyl)-N-méthyl]-amino}-méthylbenzhydrol (tel quel ou sous la forme d'un sel d'addition acide).

2. Procédé selon la revendication 1, dans lequel on isole la N-{[N-(2-hydroxyéthyl)-N-méthyl]amino}-méthylbenzophénone sous la forme de son chlorhydrate, qui est ensuite réduit.

3. Procédé selon la revendication 2, dans lequel on utilise 0,065 partie de borhydrure de sodium pour chaque partie en poids de chlorhydrate de 2-{[N-(2-hydroxyéthyl)-N-méthyl]-amino}-méthylbenzophénone.

4. 2-{[N-(2-hydroxyéthyl)-N-méthyl]-amino}-méthylbenzophénone, et ses sels d'addition acide.

5. Procédé de préparation d'un composé selon la revendication 4, consistant à faire réagir le 2-méthylaminoéthanol sur la 2-chlorométhylbenzophénone.

**Revendications pour l'Etat Contractant: AT**

1. Procédé de préparation du 2-{[N-(2-hydroxyéthyl)-N-méthyl]amino}-méthylbenzhydrol (tel quel ou sous la forme d'un sel d'addition acide), consistant à faire réagir la 2-chlorométhylbenzophénone sur le 2-méthylaminoéthanol pour donner la 2-{[N-(2-hydroxyéthyl)-N-méthyl]-amino}-méthylbenzophénone (telle quelle ou sous la forme d'un sel) et à réduire cette dernière par du borhydrure de sodium pour donner le 2-{[N-(2-hydroxyéthyl)-N-méthyl]-amino}-méthylbenzhydrol (tel quel ou sous la forme d'un sel d'addition acide).

2. Procédé selon la revendication 1, dans lequel on isole la N-{[N-(2-hydroxyéthyl)-N-méthyl]amino}-méthylbenzophénone sous la forme de son chlorhydrate, qui est ensuite réduit.

3. Procédé selon la revendication 2, dans lequel on utilise 0,065 partie de borhydrure de sodium pour chaque partie en poids de chlorhydrate de 2-{[N-(2-hydroxyéthyl)-N-méthyl]-amino}-méthylbenzophénone.

4. Procédé de préparation de la 2-{[N-(2-hydroxyéthyl)-N-méthyl]-amino}-méthylbenzophénone (telle quelle ou en tant que sel d'addition acide), consistant à faire réagir du 2-méthylaminoéthanol sur la 2-chlorométhylbenzophénone.

**Patentansprüche für die Vertragsstaaten: BE CH LI DE FR GB IT LU NL SE**

1. Verfahren zur Herstellung von 2-{[N-(2-Hydroxyethyl)-N-methyl]amino}-methylbenzhydrol (als solches oder als Säureadditionssalz, dadurch gekennzeichnet, dass man 2-Chloromethylbenzophenon mit 2-Methylaminoethanol unter Erhalt von 2-{[N-(2-Hydroxyethyl)-N-methyl]-amino}-methylbenzophenon (als solches oder als Salz) umsetzt und die letztere Verbindung mit Natriumborhydrid unter Erhalt von 2-{[N-(2-Hydroxyethyl)-N-methyl]-amino}-methylbenzhydrol (als solches oder als Säureadditionssalz) reduziert.

2. Verfahren gemäss Anspruch 1, dadurch gekennzeichnet, dass man N-{[N-(2-Hydroxyethyl)-N-methyl]-amino}-methylbenzophenol als Hydrochlorid, das dann reduziert wird, isoliert.

3. Verfahren gemäss Anspruch 2, dadurch gekennzeichnet, dass man 0,065 Teile Natriumborhydrid pro Gewichtsteil 2-{[N-(2-Hydroxyethyl)-N-methyl]-amino}-methylbenzophenon-hydrochlorid verwendet.

4. 2-{[N-(2-Hydroxyethyl)-N-methyl]-amino}-methylbenzophenon und dessen Säureadditionssalze.

5. Verfahren zur Herstellung einer Verbindung gemäss Anspruch 4, dadurch gekennzeichnet, dass man 2-Methyl-amino-ethanol mit 2-Chloromethylbenzophenon umsetzt.

**Patentansprüche für den Vertragsstaat: AT**

1. Verfahren zur Herstellung von 2-{[N-(2-Hydroxyethyl)-N-methyl]-amino}-methylbenzhydrol (als solches oder als Säureadditionssalz), dadurch gekennzeichnet, dass man 2-Chloromethylbenzophenon mit 2-Methylaminoethanol unter Erhalt von 2-{[N-(2-Hydroxyethyl)-N-methyl]-amino}-methylbenzophenon (als solches oder als Salz) umsetzt und die letztere Verbindung mit Natriumborhydrid unter Erhalt von 2-{[N-(2-Hydroxyethyl)-N-methyl]-amino}-methylbenzhydrol (als solches oder als Säureadditionssalz) reduziert.

2. Verfahren gemäss Anspruch 1, dadurch gekennzeichnet, dass man N-{[N-(2-Hydroxyethyl)-N-methyl]-amino}-methylbenzophenon als Hydrochlorid, das dann reduziert wird, isoliert.

3. Verfahren gemäss Anspruch 2, dadurch gekennzeichnet, dass man 0,065 Teile Natriumborhydrid pro Gewichtsteil 2-{[N-(2-Hydroxyethyl)-N-methyl]-amino}-methylbenzophenon-hydrochlorid verwendet.

4. Verfahren zur Herstellung von 2-{[N-(2-Hydroxyethyl)-N-methyl]-amino}-methylbenzophenon (als solches oder als Säureadditionssalz), dadurch gekennzeichnet, dass man 2-Methyl-amino-ethanol mit 2-Chloromethylbenzophenon umsetzt.